# EUROPEAN PATENT SPECIFICATION

(11) **EP 1 608 977 B1**
(45) Date of publication and mention of the grant of the patent: **15.08.2007**
(21) Application number: 04725404.0
(22) Date of filing: 02.04.2004
(51) Int. Cl.: G01N 33/543, G01N 33/58

(54) **NANOPARTICLE FOR BIOAFFINITY ASSAYS**
NANOPARTIKEL FÜR BIOAFFINITÄTSTESTS
NANOPARTICULE POUR ESSAIS DE BIOAFFINITE

(30) Priority: 02.04.2003 US 459374 P; 24.04.2003 FI 20030615
(43) Date of publication of application: 28.12.2005
(73) Proprietor: Turun Yliopisto, 20014 Turun Yliopisto (FI)
(72) Inventor: Soukka, Tero, 20540 Turku (FI); Korpimäki, Teemu, 20100 Turku (FI); Lamminmäki, Urpo, 20500 Turku (FI); Virta, Marko, 20100 Turku (FI)
(74) Representative: Maskula, Silla Marjatta
(86) International application number: PCT/FI2004/000204
(87) International publication number: WO 2004/088313

(56) References cited:
- EP-A- 0 354 847
- EP-A- 1 262 489
- WO-A-03/094849
- US-A- 5 358 722
- LUZZAGO A ET AL: "ISOLATION OF POINT MUTATIONS THAT AFFECT THE FOLDING OF THE H CHAIN OF HUMAN FERRITIN IN ESCHERICHIA-COLI" EMBO (EUROPEAN MOLECULAR BIOLOGY ORGANIZATION) JOURNAL, vol. 8, no. 2, 1989, pages 569-576, XP002286331 ISSN: 0261-4189
- HAATAJA SAULI ET AL: "Expression, purification and crystallization of Dpr, a ferritin-like protein from the Gram-positive meningitis-associated bacterium Streptococcus suis" ACTA CRYSTALLOGRAPHICA SECTION D BIOLOGICAL CRYSTALLOGRAPHY, vol. 58, no. 10 Part 2, October 2002 (2002-10), pages 1851-1853, XP008032370 ISSN: 0907-4449
- RUCKER PAUL ET AL: "Recombinant ferritin: Modulation of subunit stoichiometry in bacterial expression systems" PROTEIN ENGINEERING, vol. 10, no. 8, August 1997 (1997-08), pages 967-973, XP002286332 ISSN: 0269-2139

### SEQUENCE LISTING

<110> Soukka, Tero
   Korpimäki, Teemu
   Lamminmäki, Urpo
   Virta, Marko
<120> Nanoparticle for Bioaffinity Assays
<130> AP101838
<160> 10
<170> PatentIn version 3.1
<210> 1
   <211> 34
   <212> DNA
   <213> Artificial Sequence
<220>
   <223> primer
<400> 1
   aaggatccca tatgaacctc tgtaaccatt tcag 34
<210> 2
   <211> 31
   <212> DNA
   <213> Artificial Sequence
<220>
   <223> primer
<400> 2
   aaccatggca tatggtgaca acttacaaac t 31
<210> 3
   <211> 20
   <212> DNA
   <213> Artificial Sequence
<220>
   <223> primer
<400> 3
   gttatatcaa ctgtaaaagt 20
<210> 4
   <211> 31
   <212> DNA
   <213> Artificial Sequence
<220>
   <223> primer
<400> 4
   aaccatggca tatggaaatt gtgctcaccc a 31
<210> 5
   <211> 25
   <212> DNA
   <213> Artificial Sequence
<220>
   <223> primer
<400> 5
   gaattcggat ccttagtcgt gcttg 25
<210> 6
   <211> 72
   <212> DNA
   <213> Artificial Sequence
<220>
   <223> primer
<400> 6
   ctgctgcgaa ccgtttcaag aaaatcagct cttccggtgc tggcggtatg agctcccaga 60
   ttcgtcagaa tt 72
<210> 7
   <211> 25
   <212> DNA
   <213> Artificial Sequence
<220>
   <223> primer
<400> 7
   gaattcggat ccttagtcgt gcttg 25
<1210> 8
   <211> 73
   <212> DNA
   <213> Artificial Sequence
<220>
   <223> primer
<400> 8
<210> 9
   <211> 31
   <212> DNA
   <213> Artificial Sequence
<220>
   <223> primer
<400> 9
   aaccatggca tatggaagcg ccagcagcag c 31
<210> 10
   <211> 40
   <212> DNA
   <213> Artificial Sequence
<220>
   <223> primer
<400> 10
   agccgctggt cgtcatcgag ggtggccata tgcgttgcaa 40

## Description

### FIELD OF INVENTION

This invention relates to nanoparticles for bioaffinity assays. More specifically this invention relates to ferritin particles for bioaffinity assays.

### BACKGROUND OF THE INVENTION

Ferritin is a protein that is produced by e.g. bacteria, plants and mammals including humans. It is a particular and hollow protein consisting of multiple subunits that may be of different or similar type. Typically, a ferritin molecule consists of 24 subunits which self-assemble to a spherical structure. For example, human liver ferritin consists of heavy subunits (molecular weight 21 kDa) and light subunits (19 kDa) [Boyd D, Vecoli C, et. al. (1985) Journal of Biological Chemistry 260:11755-61]. It has diameter of 12 nm and an inside cavity with diameter of 8 nm. The inside cavity is capable of storing about 4500 ions of iron as hydrous ferric oxide [Chasteen ND, Harrison PM. (1999) Journal of Structural Biology 126:182-94]

In addition, ferritin-like spherical proteins with smaller size have been found in bacteria. Examples are Dpr protein produced by *Streptococci* [Haataja S, Penttinen A, et. al. Acta Crystallographica D (2002) 58:1851-1853], Dps protein produced by *Listeria* [Bozzi M, Mignogna G, et. al. (1997) Journal of Biological Chemistry 272:3259-3265], Dps protein produced by *Helicobacteria* [Tonello F, Dundon WG, et. al. (1999) Molecular Microbiology 34:238-246] and Dps protein produced by *Escherichia* [IIari A, Ceci P, et. al. (2002) Journal of Biological Chemistry 277:37619-37623].

The iron core is visible in electron microscopy and it has been utilised in electron microscopy as a label [Anderson KL. (1998) Biotechnic and Histochemistry 73:278-88].

Ferritin has been conjugated with other molecules chemically [Hsu KC. (1981) Scanning electron microscopy 4:17-26] or by protein fusions [Lofdahl, S, Uhlen, M et. al. US Pat 5,100,788].

Expression of foreign proteins or peptides on the surface of viruses as virus surface protein fusions has been widely studied. The display of proteins and peptides on the surface of bacteriophages T4, T7 and λ have also been studied [Sternberg, N. & Hoess, R. (1995), Proc. Natl. Acad. Sci. USA 92, 1609-1613; Ren, Z. & Black, L. (1998), Gene 215, 439-444; Danner, S. & Belasco, J. (2001), Proc. Natl. Acad. Sci. USA 98, 12954-12959]. In these examples phages connect the binding activity located on the suface of a particle to genetic information located inside the particle. Moreover, there has been research on the expression of foreign proteins on the surface of viruses with the goal of using these modified viruses as vaccines, immunogens and coating agents (US 5,008,373; US 5,041,385; US 5,463,024; US 5,736,368; US 5,804,196 and US 6,051,410). Furthermore, the use of viral or phage based capsids as functional particles in bioaffinity assays has been suggested (US 2002/0025515).

Advanges of phage and viral capsids are that they self-assemble into particle like structures. They have, however disadvantages: They are replicative in their respective hosts. They can infect their hosts by accident and accordingly result in uncontrolled outbreak of viruses or phages. They also contain nucleic acid molecules that may result in accidental production of unwanted proteins in unforeseen situations. The size of even the smallest bacteriophage is larger than optimal for optimal colloidal stability of the particles.

A marker is a molecule, which is possible to detect by chemical or physical means. Marker may have catalytic activity, which is used for the detection. Examples of those markers are nucleic acids or proteins that have catalytic activity. A useful way to detect a marker is based on its fluorescence, luminescence, optical, electric or magnetic properties. Marker proteins are widely used in biological research and especially in bioaffinity assays. The following enzymes are examples of widely used marker proteins: alkaline phosphatase (EC 3.1.3.1), β-Galactosidase (EC 3.2.1.23), β-Glucuronidase (EC 3.2.1.31), glucose oxidase (EC 1.1.3.4), luciferase (EC 1.13.12.7) and horseradish peroxidase (EC 1.11.1.7). The common feature with all of them is that their detection is possible at low concentrations by using a simple protocol. In addition to enzymes, alternative marker proteins have been described, such as fluorescent proteins [Heim R, and Tsien RY, (1996), Current Biology 6: 178-82] or coloured proteins [Lukyanov KA, Fradkov AF, et. al. (2000), Journal of Biological Chemistry 275: 25879-82].

Numerous well-defined conjugates between a binding molecule and a marker protein for assay development have been produced. A conjugate is traditionally produced by in vitro labelling of binding molecule with marker protein or peptide [Kopetzki, E.; Lehnert, K; Buckel, P. Clin. Chem. (1994), 40: 688-704]. An alternative way to produce the conjugate is to fuse genes encoding a binding molecule and a reporter protein, which results in the production of a fusion protein having both binding and marker activity [Zenno and Inouye, Biochemical and Biophysical Research Communications (1990),171:169-74].

Luzzago et al. [The EMBO Journal 8, 569-576 (1989)] discloses isolation of point mutations affecting the folding of the H chain of human ferritin in *E. Coli* using a recombinant hybrid molecule comprising the α-peptide of β-gatactosidase. If the mutations did not affect folding the α-peptide domain is segregated inside the apoferritin shell upon assembly and is unable to interact with the substrate and perform its enzymic function.

US 5,358,722 discloses ferritin analogues comprising an apoferritin protein shell surrounding a solid spherule-shaped core of inorganic or organic material which is essentially devoid of ferrihydrite. The material can be e.g. europium.

EP 0 354 847 discloses a conjugate for use in a labelling system comprising avidin or streptavidin linked to a carrier particle, e.g. apoferritin. The conjugate is useful in forming a fluorescent reagent system. The fluorescent labelling system is particularly useful in binding assays.

WO 03/094849 discloses ferritin fusion proteins for use in vaccines and other applications.

### OBJECT AND SUMMARY OF THE INVENTION

One object of the present invention is to provide alternative particles for bioaffinity assays.

Another object of the present invention is to provide bioaffinity assays making use of the alternative particles.

Yet another object of the present invention is to provide improved kits for bioaffinity assays making use of the alternative particles.

Thus, this invention provides use of a nanoparticle in a ligand binding bioaffinity assay wherein said nanoparticle comprises a self-assembling shell built up of several protein and/or peptide subunits, which protein and/or peptide subunits can be of one or several different types, assembled in an organized manner to form the shell having an inner surface facing the inside and an outer surface facing the outside of said particle wherein the shell of the nanoparticle is a recombinant apoferritin particle or a recombinant apoferritin-like Dpr/Dps protein particle, wherein
a) one or several of the types of subunits have one or several genetically fused first binding moieties per type of subunit with the binding moiety facing the outside of the particle for binding of any specific ligand binding protein; and
b)
   i) the particle contains within its shell a marker and/or
   ii) one or several of the types of subunits have one or several genetically fused second binding moieties per type of subunit with the binding moiety facing the inside and/or the outside of the particle binding a marker; and
c) the marker or markers enable detection of the particle;

This invention also provides a nanoparticle, useful for ligand binding bioaffinity assays, comprising a self-assembling shell built up of several protein and/or peptide subunits, which protein and/or peptide subunits can be of one or several different types, assembled in an organized manner to form the shell having an inner surface facing the inside and an outer surface facing the outside of said particle wherein the shell of the nanopartide is a recombinant apoferritin particle or a recombinant a poferritin-like Dpr/Dps protein particle, wherein
a) one or several of the types of subunits have one or several genetically fused first binding moieties per type of subunit with the binding moiety facing the outside of the particle for binding of any specific ligand binding protein; and
b)
   i) the particle contains within its shell a marker and/or
   ii) one or several of the types of subunits have one or several genetically fusedsecond binding moieties per type of subunit with the binding moiety facing the inside and/or the outside of the particle for binding a marker selected from the group consisting of an enzyme, luminescent protein, fluorescent or coloured protein or organic molecule, and a rare earth metal, and
c) the marker or markers enables detection of the particle.

This invention also provides a bioaffinity assay using the nanoparticle.

This invention further provides a kit for a bioaffinity assay comprising the nanoparticle.

### BRIEF DESCRIPTION OF THE DRAWINGS

Figure 1 shows the structure of a protein nanoparticle.
Figure 2 shows detection of the binding activity of a protein particle.
Figure 3 shows the structure of plasmid pBccpHFI.
Figure 4 shows the structure of plasmid pPrGHFI.
Figure 5 shows the structure of plasmid pTSHscHFI.
Figure 6 shows the structure of plasmid pCBPHFI.

### DETAILED DESCRIPTION OF THE INVENTION

### Definition of terms

The terms "apoferritin" shall be understood to consist of ferritin deficient of molecules in the cavity inside of the protein.

The term "an apoferritin-like particle" shall be understood to consist of a ferritin-like particle deficient of molecules in the cavity inside of the protein, as defined in the claims. The "apoferritin-like particle" typically is a self-assembling particle of protein consisting of a specific number of subunits. The particle is typically spherical. Examples of ferritin-like particles have been given above.

The term "bioaffinity assays" shall be understood to include one-step and multistep competitive and non-competitive ligand binding assays and immunoassays based on a single or multiple specific ligand binding moieties, e.g. monoclonal antibodies, polypeptides, receptors, recombinant antibodies or antibody fragments as well as artificial binders like aptamers and engineered proteins.

Bioaffinity assays include heterogeneous and homogenous assays. In heterogeneous assay the analyte is bound to a solid phase and particle is used for the detection of bound analyte. A particle can bind directly to the analyte or to a molecule, which is bound to the analyte. Particle and analyte can be added to reaction either sequentially or simultaneously. In homogenous assay the analyte is detected from solution without the separation of unbound particle. Homogenous assay can be based on for example Fluorescence Resonance Energy Transfer (FRET) or Bioluminescence Resonance Energy Transfer (BRET) (Boute, N, Jockers, R and Issad, T. 2002. Trends in Pharmacological Sciences 23:351-354). It can also be based on channeling of substrate, product or intermediate of an enzyme reaction (Gibbons, I, Armenta, R, DiNello, RK and Ullman, EF. 1987. Methods in Enzymology 136:93-103).

The term "marker" shall be understood as a feature detectable by measuring luminescence or absorbance, as well as other optical properties, electrical properties e.g. electrical current or voltage or magnetic properties, originating directly or resulted indirectly from the existence of the feature. Example of direct measurement of the feature is measurement of fluorescence emission of green fluorescent protein using appropriate excitation light. Example of indirect measurement of the feature is measurement of luminescence originating from a chemical reaction catalysed by luciferase enzyme marker. Example of measurement of electrical properties is e.g. detection of redox-reaction by measuring electrical voltage or current.

The term "luminescence" shall be understood to cover luminescence, bioluminescence, chemiluminescence, electroluminescence, photoluminescence, fluorescence, delayed fluorescence and phosphorescence.

The term "luminescent protein" and ''fluorescent protein", respectively, shall be understood as a protein or enzyme, which produces luminescence or is fluorescent, respectively, with or without prosthetic groups. Example of luminescent protein is luciferase enzyme. Example of fluorescent protein is green fluorescent protein (GFP).

The term "nanoparticle" shall be understood as a particulate reagent composed of multiple "subunits", each composed of proteins or polypeptides and, in addition, optionally of single or multiple features of the following: nucleic acids, prosthetic groups, organic and inorganic compounds. The particulate has at least a single "binding moiety" on the outer surface and the particulate contains a single "marker" or multiple "markers". Dimensions of the particulate are between one nanometer and ten micrometers.

The term "lanthanide" and "rare earth metal" shall be understood to include elements and combinations of different elements of rare earth ions from the following: neodymium (Nd), samarium (Sm), europium (Eu), gadolinium (Gd), terbium (Tb), dysprosium (Dy), Erbium (Er), ytterbium (Yb) and yttrium (Y).

The term "subunit" shall be understood as a single protein or polypeptide or complex of multiple proteins or polypeptides, composed of identical or different components.

The term "binding moiety" shall be understood to cover monoclonal antibodies, polypeptides, receptors, recombinant antibodies or antibody fragments as well as artificial binders like aptamers and engineered proteins, or derivatised form of any of the listed features. Example of polypeptide is calmodulin binding peptide (CBP). Example of derivatised feature is a peptide sequence of biotin carboxyl carrier protein (BCCP), which can be biotinylated in vivo with BirA biotin ligase enzyme.

The term "enzyme" shall be understood a protein or polypeptide or nucleic acid with catalytical activity. Examples of enzymes are lusiferase and galactose oxidase (GAO).

The term "galactose oxidase (GAO)" shall be understood as enzyme with Enzyme Commision number EC 1.1.3.9.

The term "colored protein" shall be understood as a protein or polypeptide, which has a significant absorption at visible wavelengths, 300 - 700 nm, with or without prosthetic groups.

The term "organic molecule" shall be understood as any chemical compound containing at least carbon with molecular weight below 7000 Dalton. Examples of organic molecules are prosthetic groups in fluorescent allophycocyanin protein.

The term "inorganic molecule" shall be understood as any inorganic atom, chemical compound composed of inorganic atoms or combination of atoms in an organized manner. Example of inorganic molecule is fluorescent CdSe semiconductor particle.

The term "self-assembling shell" shall be understood as a particulate structure capable of assembling itself from a pool of vital shell proteins.

The term "vital shell protein" shall be understood as protein which is needed for the self-assembly of a particulate entity e.g. nanoparticle.

The term "GFP" shall be understood as green fluorescent protein from *Aequorea victoria,* its mutant derivatives or homologous protein from other species.

The term "CRP" shall be understood as C-reactive protein.

The term "TSH" shall be understood as thyroid stimulating hormone.

The term "BCCP" shall be understood as Biotin carboxyl carrier protein.

The term "Protein G" shall be understood as Protein G from bacteria in genera *Streptococcus.*

The term "Protein A" shall be understood as Protein A from bacteria in genera *Staphylococcus.*

The term "Protein L" shall be understood as Protein L from bacteria in genera *Peptostreptococcus.*

### General description of preferred embodiments

The nanoparticles according to the invention can have useful properties. These can be, but are not limited to, low cost, simple production, high stability and highly defined structure. Production can be very simple whereas a simple microbial fermentation with minor down-stream processing is typically all that is needed.

The nanoparticle according to the invention can have a marker that is an enzyme, luminescent protein, fluorescent or coloured protein or organic molecule, or a rare earth metal. If the marker is a protein, it can be an enzyme such as luciferase or GAO, or a fluorescent protein like GFP. If the marker is a rare earth metal ion, it can be a Tb, Eu, Sm or Dy ion. The marker can also be an inorganic particle, e.g. a CdSe particle.

The nanoparticle of the invention can have, in addition to the first and second binding moieties third binding moieties. One or several of the types of subunits can e.g. have one or several third binding moieties per type of subunit with the binding moiety facing the outside of the particle for binding to a solid support. The nanoparticle can also have additional binding moieties with additional functions.

The shell of the nanoparticle is a recombinant apoferritin or a ferritin-like particle.

The first, second, third or additional binding moiety can be protein A, protein G, protein L or calmodulin binding peptide (CBP). The first, second, third or additional binding moiety can be an antibody against e.g. CRP, ABO blood group antigens or TSH. The first, second, third or additional binding moiety can be protein A, protein G, protein L or CBP.

The minimum radius of the nanoparticle is typically from 10 to 40 nm. The number of subunits of the shell of the nanoparticle is typically more than 8, preferably more than 20.

Apoferritin has a size that results in enhanced colloidal stability. It consists only protein subunits without nucleic acids, which makes it a biosafe particle as such and it also self-assembles in solution.

### Description of the figures

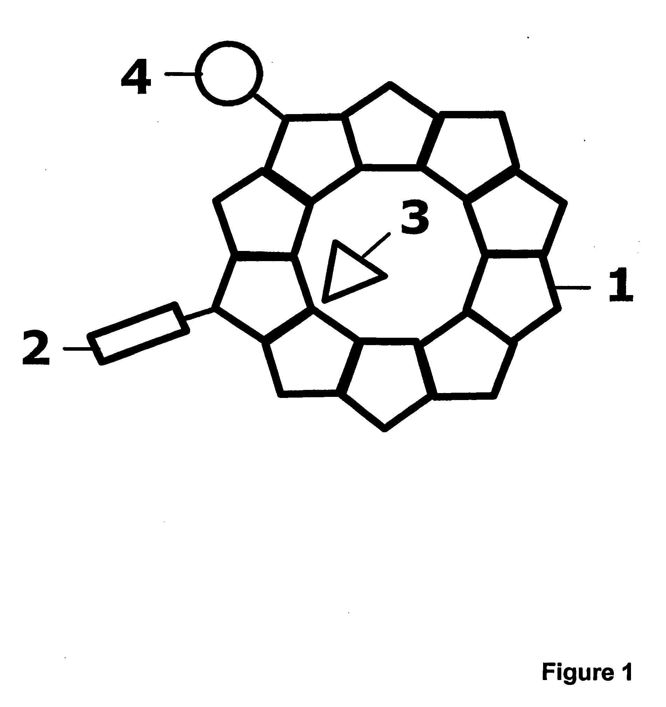

Figure 1 shows the structure of a protein nanoparticle according to the invention. The figure shows a protein shell 1, a first binding molecule 2 facing the outside of the nanoparticle, a marker 3 within the shell of the nanoparticle and a second or third binding molecule 4 facing the outside of the nanoparticle.

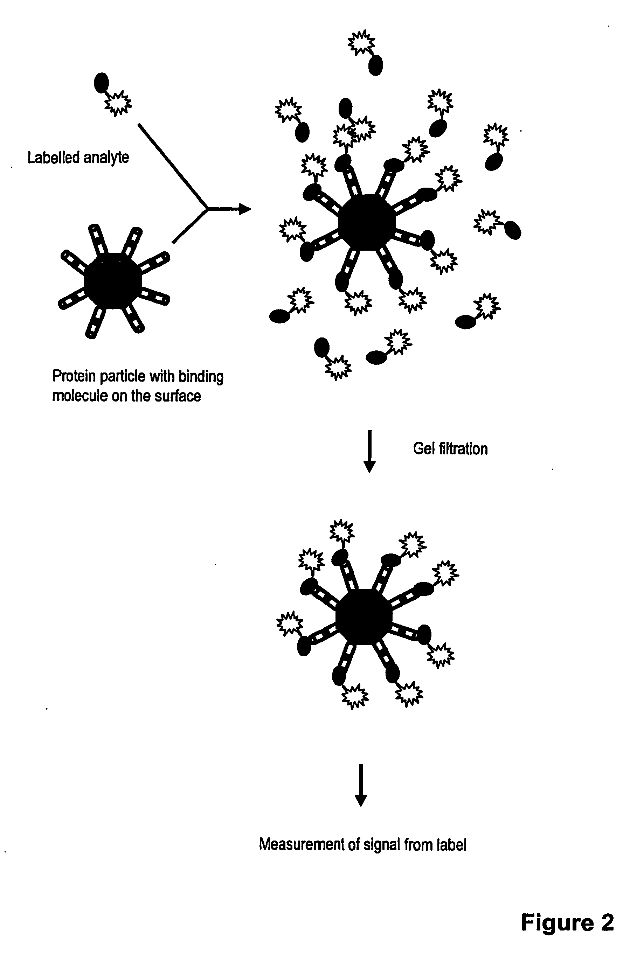

Figure 2 shows detection of binding activity of a protein particle. Analyte specific to the binding molecule to be tested is labelled with a molecule suitable for detection. Labelled analyte is then reacted with particles and particle/analyte complexes are separated from unbound analyte by gel filtration and the signal of the label is measured.

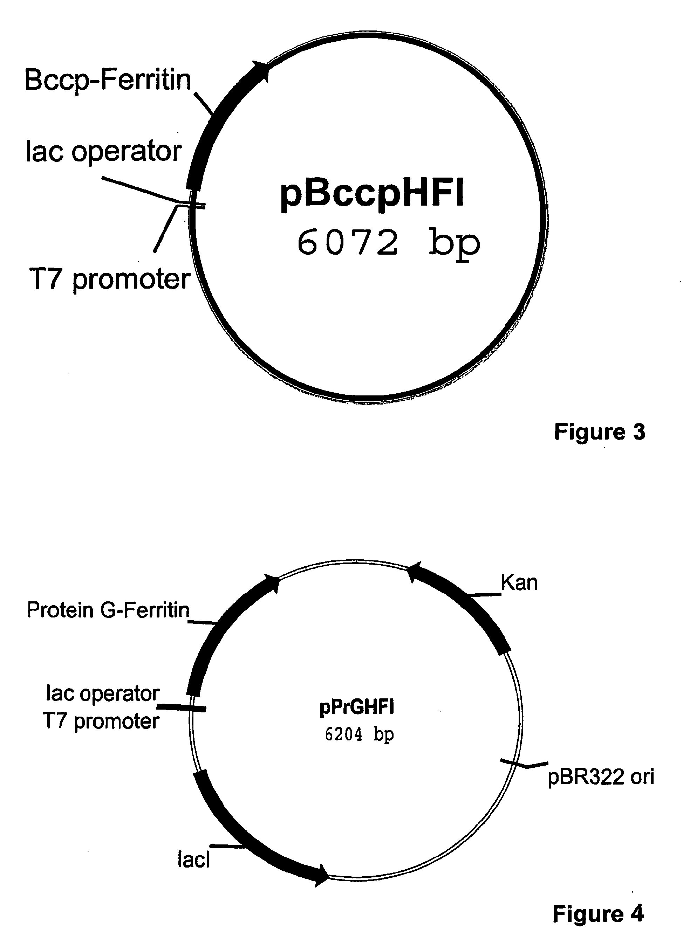

Figure 3 shows the structure of plasmid pBccpHFI. Abbreviations stand for: Bccp-ferritin = gene encoding BCCP-Human ferritin light chain fusion protein.
Figure 4 shows the structure of plasmid pPrGHFI. Abbreviations stand for: pBR322 ori = Origin of replication, Kan = kanamycin resistance gene, Lacl = gene encoding Lac repressor, Protein G-ferritin = gene encoding Protein G-Human ferritin light chain fusion protein.

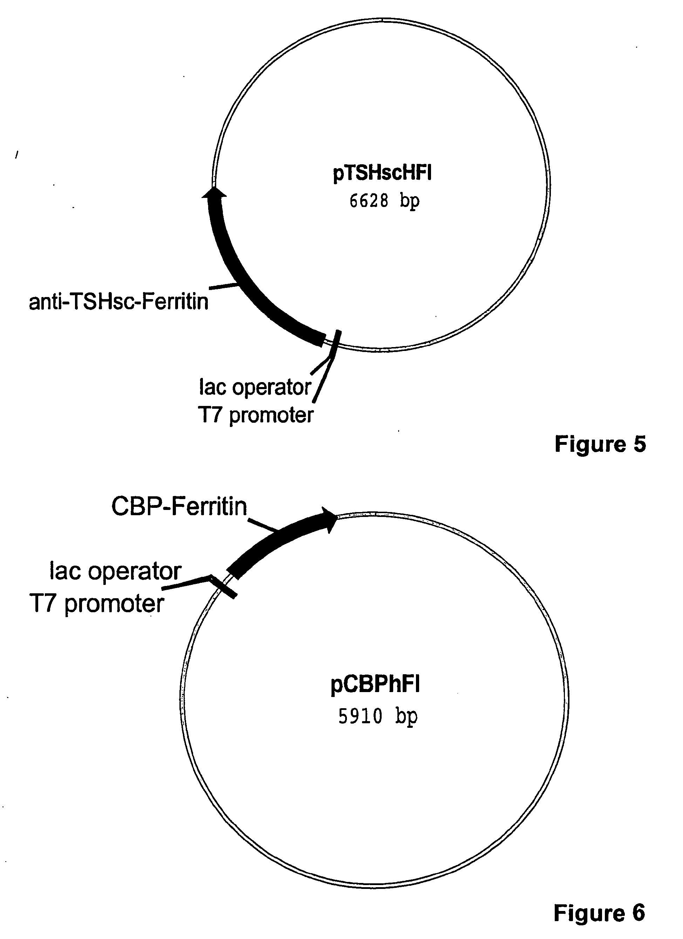

Figure 5 shows the structure of plasmid pTSHscHFI. Abbreviations stand for: anti-TSHsc-ferritin = gene encoding antiTSHscFv antibody-Human ferritin light chain fusion protein.
Figure 6 shows the structure of plasmid pCBPHFI. Abbreviations stand for: CBP-ferritin = gene encoding CBP-Human ferritin light chain fusion protein.

### Methods

### DNA manipulations

All DNA manipulations were made according to known protocols [Sambrook J, Fritsch EF, and Maniatis T, (1989), Molecular Cloning: A laboratory manual, Cold Spring Harbour Laboratory Press, Cold Spring Harbour),

### Bacteria strains

The following bacterial strains are used in the examples: XL-1 Blue, BL21, BL21 (DE3), BL21 (DE3:pLysS), Origami B, Origami B(DE3) and Origami B(DE3:pLysS) (Stratagene, La Jolla, CA, USA).

### Measurement of fluorescence

Time-resolved fluorescence of europium and terbium was measured with Delfia reagents from PerkinElmer Life Sciences (Boston, MA, USA). Fluorescence of Alexa Fluor 594 (Molecular Probes Europe, Leiden, The Netherlands) was measured as suggested by the manufacturer. All measurements were done with Wallac Victor multilabel counter (Perkin Elmer Life Sciences, Boston, MA, USA).

### Detection of reporter proteins

The activity of firefly luciferase was determined by measuring luminescence produced with Luciferase Assay Kit from BioThema ltd (Haninge, Sweden).

The activity of galactose oxidase was determined by measuring luminescence from luminol oxidation by H₂O₂ generated in the reaction of enzyme with 50 mM galactose in 100 mM phosphate buffer containing 1 mM luminol and 0.4 mM CuSO₄ at pH 8.6. All measurements were done with Wallac Victor multilabel counter (Perkin Elmer Life Sciences, Boston, MA, USA).

### Example 1

### Production of ferritin based particles

*Escherichia coli* cells expressing plasmids encoding a binding molecule fused to N-terminus of ferritin (Example 8, Example 9, Example 10 and Example 11) subunits were grown in 50 ml of SB medium in shaking at 37°C until OD₆₀₀ reached 0.4. The protein production was induced by adding IPTG to the concentration of 0.5 mM and the cultivation was continued at 26 °C over night. After that the cells were collected by centrifugation at 1500 g for 10 minutes and suspended to 5 ml of phosphate-buffered saline (PBS). The cells were then lysed by sonication and cell debris removed by centrifugation at 5000 g for 10 minutes. Supernatant was then filtered with 100 kDa cut-off value filters (Pall Life Science, Ann Arbor, MI, USA) and retentate was suspended to PBS.

### Example 2

### Purification of the particles with gel filtration

Protein nanoparticles were purified by gel filtration with 10 ml Sepharose 6B column (Amersham Biosciences Corp, Piscataway, NJ, USA). The column was first equilibrated by using buffer containing 5 mM Tris-HCI, 0.01 % Tween-20 and 0.05 % NaN₃ at pH 7.5 with 10 volumes of the column. A 500 µl sample was applied to the column and 1 ml fractions were collected.

### Example 3

### Detection of binding activity on the surface of protein particle by using gel filtration

Rationale of the analysis of the activity of binding molecules on the surfaces of the particles is shown in Figure 2. Analyte specific to the binding molecule to be tested was labelled with a molecule suitable for detection. Analytes were labelled as follows: TSH (thyroid stimulating hormone) (Scripps Laboratories, San Diego, CA, USA), streptavidin (Perkin Elmer Life Sciences, Boston, MA, USA) and antibodies with Europium and Calmodulin with Alexa Fluor 594. Mixture of four monoclonal Eu-labelled antibodies was used in the analysis of Protein G activity. Labelling of the molecules with Europium was done with a reagent kit obtained from Perkin Elmer Life Sciences, (Boston, MA, USA). Labelled calmodulin was obtained from Molecular Probes Leiden, The Netherlands). Labelled analyte was reacted with particles and particle/analyte complexes were separated from unbound analyte by gel filtration as described in Example 2. Ferritin based particles were produced as described in Example 1. Particles produced without binding molecule were used as negative control.

### Example 4

### Detection of binding activity on the surface of protein Particle by using multiwell plates

In vitro biotinylation of TSH and antibodies was done by reagent kit obtained from Perkin Elmer Life Sciences, Boston, MA, USA). Mixture of four monoclonal Eu-labelled antibodies was used in the analysis of Protein G activity. Biotinylated molecules were attached to streptavidin-coated 96 well plates (Innotrac Diagnostics, Turku, Finland) as described in the kit and wells were washed four times. After that the particles to be tested were added and the wells were again washed for four times. Then the analyte labelled as described in Example 3 was added, wells washed four times and signal from the label measured.

### Example 5

### Detection of binding activity on the surface of protein particle by using molecular weight cut-off filters Analyte molecules were labelled as described in Example 3. Particles together with bound analyte were separated from smaller molecules with 100 kDa cut-off value filters (Pall Life Science, Ann Arbor, MI, USA). Signal of the labelled molecule was measured from the retentate.

### Example 6

### Loading of ferritin based particles with terbium

The reaction buffer used in the loading of terbium ions into ferritin consisted of 50 mM HEPES, 50 mM NaCl and 10 mM TbCl₃ at pH 7.0. Ferritin was added to the buffer to 0.1 µM and the reaction was incubated at 37 °C for 20 h. Unreacted terbium was removed by gel filtration with NAP-5 column (Amersham Biosciences Corp, Piscataway, NJ, USA).

### Example 7

### Loading of ferritin with europium.

Pelleted fraction of a 100 ml of bacterial culture produced according to Example 1 by using *E. coli* BL21 (DE3:pLysS:pBccpHFI) was dissolved to 1.5 ml of 8 M urea and centrifuged at 5000 g. 10 ml of solution containing 100 mM Tris, 150 mM NaCl at pH 8.5 was added and the mixture was incubated at +4 °C over night. HCl was then added until pH of the solution reached 6.0 and the solution was incubated at 4 °C over night. 0,056 ml of 0.01 M EuCl₃ solution was added and solution was incubated at 25 °C for 2 h after which buffer (133 mM Tris, 150 mM NaCl, pH 9.0) was gradually added until pH reached 8.5. Precipitated europium was then separated from soluble europium-loaded ferritin by collecting supernatant after 10 min centrifugation at 5000 g. Particles loaded with europium were tested for the binding capacity according to Example 4 and the europium inside ferritin was detected. The reaction containing excess of soluble biotin (0.001 mM biotin) was used as control. Signal without biotin was 163 fold as compared to control showing the binding of Eu labelled particles to streptavidin coated solid phase.

### Example 8

### Production of Protein G on the surface of ferritin

Gene encoding Streptococcal Protein G was inserted to plasmid producing human ferritin light chain by ligating Nhel digested fragment obtained by PCR with oligonucleotides 5'-AAGGATCCCATATGAACCTCTGTAACCATTTCAG (SEQ ID NO: 1) and 5'-AACCATGGCATATGGTGACAACTTACAAACT (SEQ ID NO: 2) with Streptococcus G148 genomic DNA as template with Nhel digested plasmid pET-26(+)rHuLFt (Grace JE Jr, Van Eden ME, Aust SD. 2000. Archives in Biochemistry and Biophysics 384:116-22). The resulting construct was transformed to *E. coli* BL21 (DE3:pLysS) cells. Structure of the resulting plasmid, pPrGHFI, was verified by partial sequencing. The structure of pPrGHFI is shown in Figure 4. Ferritin based particles expressing Protein G on their surface were produced with *E. coli* BL21 (DE3:pLysS:pPrGHFI) cells by using protocol described in Example 1. The retentate was suspended to 1 ml and diluted tenfold prior to the analysis of the functionality according to Example 5 using Eu-labelled antibodies as label. The particles with Protein G gave 5.5 fold signal as compared to particles produced with pET-26(+)rHuLFt.

### Example 9

### Production of scfv fragment on the surface of ferritin

Gene encoding anti-TSHscFv fragment was inserted to plasmid producing human ferritin light chain by ligating Nhel digested fragment obtained by PCR with oligonucleotides 5'-GTTATATCAACTGTAAAAGT (SEQ ID NO: 3) and 5'-AAC-CATGGCATATGGAAATTGTGCTCACCCA (SEQ ID NO: 4) with pTSHscHoc as template with Nhel degested plasmid pET-26(+)rHuLFt (Grace JE Jr, Van Eden ME, Aust SD. 2000. Archives in Biochemistry Biophysics 384:116-22). The resulting construct was transformed to *E.* coli Origami B(DE3:pLysS) cells. Structure of the resulting plasmid, pTSHscHFI, was verified by partial sequencing. The structure of pTSHscHFI is shown in Figure 5. Ferritin based particles expressing anti-TSHscFv antibody on their surface were produced with *E. coli* Origami B(DE3:pLysS:pTSHHFI) cells by using protocol described in Example 1. The retentate was suspended to 1 ml and diluted fivefold prior to the analysis of the functionality according to Example 5 using Eu-labelled TSH as label. The particles with anti-TSHscFv gave 33-fold signal as compared to particles produced with pET-26(+)rHuLFt.

### Example 10

### Production of Calmodulin Binding Peptide on the surface of ferritin

Gene encodin cbp was constructed in two PCR reactions. First PCR was done with oligonucleotides 5'-GAATTCGGATCCTTAGTCGTGCTTG (SEQ ID NO: 5) and 5'-CTGCTGCGAACCGTTTCAAGAAAATCAGCTCTTCCGGTGCTGGCG-GTATGAGCTCCCAGATTCGTCAGAATT (SEQ ID NO: 6) with pET-26(+)rHuLFt as template. The product of the first PCR was the used as template of the second PCR with oligonucleotides 5'-GAATTCGGATCCTTAGTCGTGCTTG (SEQ ID NO: 7) and 5'-TAGATATACATATGAAACGCCGTTGGAAGAAAGCGTTCATCG-CTGTTTCTGCTGCGAACCGTTTCAAGAAAAT (SEQ ID NO: 8). The Ndel-BamHI digested second product was ligated with 5.3 kb Ndel-BamHI fragment of pET-26(+)rHuLFt. The resulting construct was transformed to *E. coli* BL21(DE3:pLysS) cells. Structure of the resulting plasmid, pCBPHFI, was verified by partial sequencing. The structure of pCBPHFI is shown in Figure 6. Ferritin based particles expressing cbp on their surface were produced with *E. coli* BL21 (DE3:pLysS:pCBPHFI) cells by using protocol described in Example 1. The retentate was suspended to 1 ml and diluted five-fold prior to the analysis of the functionality according to Example 3 with Alexa Fluor 594 labelled calmodulin. The particles with cbp gave 4.1-fold signal as compared to particles produced with pET-26(+)rHuLFt.

### Example 11

### Production of Biotinylated Peptide on the surface of ferritin

Gene encoding Biotin Carboxyl Carrier Peptide (BCCP) was inserted to plasmid producing human ferritin light chain by ligating Nhel digested fragment obtained by PCR with oligonucleotides 5'-AACCATGGCATATGGAAGCGCCAGCAGCAGC (SEQ ID NO: 9) and 5'-AGCCGCTGGTCGTCATCGAGGGTGGCCATATGCGTT-GCAA (SEQ ID NO: 10) with *E. coli* XL-1 Blue genomic DNA as template with Nhel digested plasmid pET-26(+)rHuLFt (Grace JE Jr, Van Eden ME, Aust SD. 2000. Archives in Biochemistry and Biophysics 384:116-22). The resulting construct was transformed to *E. coli* BL21(DE3:pLysS) cells. Structure of the resulting plasmid, pBccpHFI, was verified by partial sequencing. The structure of pBccpHFI is shown in Figure 3. Ferritin based particles expressing BCCP on their surface were produced with *E. coli* BL21(DE3:pLysS:pBccpHFI) cells by using protocol described in Example 1. The retentate was suspended to 1 ml and diluted thousand fold prior to the analysis of the functionality according to Example 4 using Eu-labelled streptavidin as label. The particles produced with pBccpHFI gave 326 fold signal as compared to particles produced with pET-26(+)rHuLFt.

## Claims

1. Use of a nanoparticle in a ligand binding bioaffinity assay wherein said nanoparticle comprises a self-assembling shell built up of several protein and/or peptide subunits, which protein and/or peptide subunits can be of one or several different types, assembled in an organized manner to form the shell having an inner surface facing the inside and an outer surface facing the outside of said particle wherein the shell of the nanoparticle is a recombinant apoferritin particle or a recombinant apoferritin-like Dpr/Dps protein particle, wherein
a) one or several of the types of subunits have one or several genetically fused first binding moieties per type of subunit with the binding moiety facing the outside of the particle for binding of any specific ligand binding protein; and
b)
i) the particle contains within its shell a marker and/or
ii) one or several of the types of subunits have one or several genetically fused second binding moieties per type of subunit with the binding moiety facing the inside and/or the outside of the particle binding a marker; and
c) the marker or markers enable detection of the particle;

2. The use according to claim 1 **characterised in that** the shell of the nanoparticle is a Dpr/Dps protein selected from the group consisting of Dpr/Dps proteins produced by Streptococci, *Listeria, Helicobacter* and *Escherichia*.

3. The use according to claim 1 or 2 **characterised in that** the marker is selected from the group consisting of an enzyme, luminescent protein, fluorescent or coloured protein or organic molecule and a rare earth metal.

4. The use according to claim 3 **characterised in that** the marker is a protein, preferably an enzyme selected from the group consisting of luciferase, GAO and GFP.

5. The use according to claim 3 **characterised in that** the marker is a lanthanide preferably selected from the group consisting of Tb, Eu, Sm and Dy.

6. The use according to any of the preceding claims **characterised in that** one or several of the types of subunits have one or several genetically fused third binding moieties per type of subunit with the binding moiety facing the outside of the particle for binding to a solid support.

7. The use according to any of the preceding claims **characterised in that** a first binding moiety is selected from the group consisting of protein A, protein G, protein L, calmodulin binding peptide (CBP) and biotin carboxyl carrier protein (BCCP).

8. The use according to any of the preceding claims **characterised in that** a first binding moiety is an antibody against one of members of the group consisting of CRP, ABO blood group antigens and TSH.

9. The use according to any of the preceding claims **characterised in that** a second binding moiety is a binding moiety selected from the group consisting of protein A, protein G, protein L, calmodulin binding protein (CBP) and biotin carboxyl carrier protein (BCCP).

10. The use according to any of the preceding claims **characterised in that** a second binding moiety is an antibody against one of the group consisting of CRP, ABO blood group antigens and TSH.

11. The use according to any of the preceding claims **characterised in that** the minimum radius of the nanoparticle is from 10 to 40 nm.

12. The use according to any of the preceding claims **characterised in that** the number of subunits is more than 8, preferably more than 20.

13. A nanoparticle, useful for ligand binding bioaffinity assays, comprising a self-assembling shell built up of several protein and/or peptide subunits, which protein and/or peptide subunits can be of one or several different types, assembled in an organized manner to form the shell having an inner surface facing the inside and an outer surface facing the outside of said particle wherein the shell of the nanoparticle is a recombinant apoferritin particle or a recombinant apoferritin-like Dpr/Dps protein particle, wherein
a) one or several of the types of subunits have one or several genetically fused first binding moieties per type of subunit with the binding moiety facing the outside of the particle for binding of any specific ligand binding protein; and
b)
i) the particle contains within its shell a marker selected from the group consisting of an enzyme, luminescent protein, fluorescent or coloured protein or organic molecule, and a rare earth metal and/or
ii) one or several of the types of subunits have one or several genetically fused second binding moieties per type of subunit with the binding moiety facing the inside and/or the outside of the particle binding a marker selected from the group consisting of an enzyme, luminescent protein, fluorescent or coloured protein or organic molecule, and a rare earth metal; and
c) the marker or markers enable detection of the particle;

14. The nanoparticle according to claim 13 **characterised in that** the first binding moieties are selected from the group consisting of monoclonal antibodies, polypeptides, receptors, recombinant antibodies or antibody fragments, aptamers, engineered proteins, and derivatives thereof.

15. The nanoparticle according to claim 14 **characterised in that** the marker is a protein, preferably an enzyme selected from the group consisting of luciferase, GAO and GFP.

16. The nanoparticle according to claim 13 **characterised in that** the marker is a lanthanide preferably selected from the group consisting of Tb, Eu, Sm and Dy.

17. The nanoparticle according to claim 13 **characterised in that** one or several of the types of subunits have one or several third genetically fused binding moieties per type of subunit with the binding moiety facing the outside of the particle for binding to a solid support.

18. The nanoparticle according to claim 14 **characterised in that** a first binding moiety is selected from the group consisting of protein A, protein G, protein L, calmodulin binding peptide (CBP) and biotin carboxyl carrier protein (BCCP).

19. The nanoparticle according to claim **14 characterised in that** a first binding moiety is an antibody against one of members of the group consisting of CRP, ABO blood group antigens and TSH.

20. The nanoparticle according to any of claims 13 to 19 **characterised in that** a second binding moiety is a binding moiety selected from the group consisting of protein A, protein G, protein L, calmodulin binding protein (CBP) and biotin carboxyl carrier protein (BCCP).

21. The nanoparticle according to any of claims 13 to 20 **characterised in that** a second binding moiety is an antibody against one of the group consisting of CRP, ABO blood group antigens and TSH.

22. The nanoparticle according to any of claims 13 to 21 **characterised in that** the minimum radius of the nanoparticle is from 10 to 40 nm.

23. The nanoparticle according to any of claims 13 to 22 **characterised in that** the number of subunits is more than 8, preferably more than 20.

24. Kit for a ligand binding immunoassay comprising the nanoparticle according to any of claims 13 to 23.

## Patentansprüche

1. Verwendung eines Nanopartikels in einem Ligandenbindungs-Bioaffinitätsassay, wobei das Nanopartikel eine sich selbst organisierende Hülle umfasst, aufgebaut aus mehreren Protein- und/oder Peptiduntereinheiten, wobei die Protein- und/oder Peptiduntereinheiten von einem oder mehreren verschiedenen Typen sein können, die in einer organisierten Weise unter Bildung einer Hülle mit einer inneren Oberfläche, die dem Inneren zugewandt ist und einer äußeren Oberfläche, die dem Äußeren des Partikels zugewandt ist, zusammengesetzt sind, wobei die Hülle des Nanopartikels ein rekombinantes Apoferritin-Partikel oder ein rekombinantes Apoferritin-artiges Dpr/Dps-Protein-Partikel ist, wobei
a) einer oder mehrere der Typen von Untereinheiten eine oder mehrere genetisch fusionierte erste Bindungsgruppierungen pro Typ von Untereinheit hat/haben, wobei die Bindungsgruppierung dem Äußeren des Partikels zum Binden eines spezifischen Ligandenbindungsproteins zugewandt ist; und
b)
i) das Partikel innerhalb seiner Hülle einen Marker enthält und/oder
ii) einer oder mehrere Typen von Untereinheiten eine oder mehrere genetisch fusionierte zweite Bindungsgruppierungen pro Typ von Untereinheit hat/haben, wobei die Bindungsgruppierung, die dem Inneren und/oder dem Äußeren des Partikels zugewandt ist, einen Marker bindet; und
c) der Marker oder die Marker die Detektion des Partikels ermöglicht/ermöglichen.

2. Verwendung gemäß Anspruch 1, **dadurch gekennzeichnet, dass** die Hülle des Nanopartikels ein Dpr/Dps-Protein ist, ausgewählt aus der Gruppe, bestehend aus Dpr/Dps-Proteinen, die von *Streptococci, Listeria, Helicobacter* und *Escherichia* erzeugt wurden.

3. Verwendung gemäß Anspruch 1 oder 2, **dadurch gekennzeichnet, dass** der Marker ausgewählt ist aus der Gruppe, bestehend aus einem Enzym, lumineszenten Protein, fluoreszenten oder farbigen Protein oder einem organischen Molekül und einem Seltenerdmetall.

4. Verwendung gemäß Anspruch 3, **dadurch gekennzeichnet, dass** der Marker ein Protein ist, vorzugsweise ein Enzym, ausgewählt aus der Gruppe, bestehend aus Luciferase, GAO und GFP.

5. Verwendung gemäß Anspruch 3, **dadurch gekennzeichnet, dass** der Marker ein Lanthanid ist, vorzugsweise ausgewählt aus der Gruppe, bestehend aus Tb, Eu, Sm und Dy.

6. Verwendung gemäß einem der vorhergehenden Ansprüche, **dadurch gekennzeichnet, dass** einer oder mehrere der Typen von Untereinheiten eine oder mehrere genetisch fusionierte dritte Bindungsgruppierungen pro Typ von Untereinheit hat/haben, wobei die Bindungsgruppierung dem Äußeren des Partikels zum Binden an einen festen Träger zugewandt ist.

7. Verwendung gemäß einem der vorhergehenden Ansprüche, **dadurch gekennzeichnet, dass** eine erste Bindungsgruppierung ausgewählt ist aus der Gruppe, bestehend aus Protein A, Protein G, Protein L, Calmodulin-Bindungs-Peptid (CBP) und Biotin-Carboxyl-Carrier-Protein (BCCP).

8. Verwendung gemäß einem der vorhergehenden Ansprüche, **dadurch gekennzeichnet, dass** eine erste Bindungsgruppierung ein Antikörper gegen eines der Mitglieder der Gruppe, bestehend aus CRP, ABO-Blutgruppenantigenen und TSH, ist.

9. Verwendung gemäß einem der vorhergehenden Ansprüche, **dadurch gekennzeichnet, dass** eine zweite Bindungsgruppierung eine Bindungsgruppierung ist, ausgewählt aus der Gruppe, bestehend aus Protein A, Protein G, Protein L, Calmodulin-Bindungs-Protein (CBP) und Biotin-Carboxyl-Carrier-Protein (BCCP).

10. Verwendung gemäß einem der vorhergehenden Ansprüche, **dadurch gekennzeichnet, dass** eine zweite Bindungsgruppierung ein Antikörper gegen eines aus der Gruppe, bestehend aus CRP, ABO-Blutgruppenantigenen und TSH, ist.

11. Verwendung gemäß einem der vorhergehenden Ansprüche, **dadurch gekennzeichnet, dass** der minimale Radius des Nanopartikel von 10-40 Nanometer beträgt.

12. Verwendung gemäß einem der vorhergehenden Ansprüche, **dadurch gekennzeichnet, dass** die Anzahl an Untereinheiten größer als 8, vorzugsweise größer als 20, ist.

13. Nanopartikel, das für Ligandenbindungs-Bioaffinitätsassays zweckmäßig ist, umfassend eine sich selbst organisierende Hülle, aufgebaut aus mehreren Protein- und/oder Peptiduntereinheiten, wobei die Protein- und/oder Peptiduntereinheiten von einem oder mehreren verschiedenen Typen sein können, die in einer organisierten Weise unter Bildung einer Hülle mit einer inneren Oberfläche, die dem Inneren zugewandt ist, und einer äußeren Oberfläche, die dem Äußeren des Partikels zugewandt ist, zusammengesetzt sind, wobei die Hülle des Nanopartikels ein rekombinantes Apoferritin-Partikel oder ein rekombinantes Apoferritin-artiges Dpr/Dps-Protein-Partikel ist, wobei
a) einer oder mehrere der Typen von Untereinheiten eine oder mehrere genetisch fusionierte erste Bindungsgruppierungen pro Typ von Untereinheit hat/haben, wobei die Bindungsgruppierung dem Äußeren des Partikels zum Binden eines beliebigen spezifischen Ligandenbindungsproteins zugewandt ist; und
b)
i) das Partikel innerhalb seiner Hülle einen Merker enthält, ausgewählt aus der Gruppe, bestehend aus einem Enzym, lumineszenten Protein, fluoreszenten oder farbigen Protein oder organischen Molekül und einem Seltenerdmetall, und/oder
ii) einer oder mehrere der Typen von Untereinheiten eine oder mehrere genetisch fusionierte zweite Bindungsgruppierungen pro Typ von Untereinheit hat/haben, wobei die Bindungsgruppierung, die dem Inneren und/oder dem Äußeren des Partikels zugewandt ist, einen Marker bindet, ausgewählt aus der Gruppe, bestehend aus einem Enzym, lumineszenten Protein, fluoreszenten oder farbigen Protein oder organischen Molekül und einem Seltenerdmetall; und
c) der Marker oder die Marker die Detektion des Partikels ermöglicht/ermöglichen.

14. Nanopartikel gemäß Anspruch 13, **dadurch gekennzeichnet, dass** die ersten Bindungsgruppierungen ausgewählt sind aus der Gruppe, bestehend aus monoklonalen Antikörpern, Polypeptiden, Rezeptoren, rekombinanten Antikörpern oder Antikörperfragmenten, Aptameren, manipulierten Proteinen und Derivaten davon.

15. Nanopartikel gemäß Anspruch 14, **dadurch gekennzeichnet, dass** der Marker ein Protein ist, vorzugsweise ein Enzym, ausgewählt aus der Gruppe, bestehend aus Luciferase, GAO und GFP.

16. Nanopartikel gemäß Anspruch 13, **dadurch gekennzeichnet, dass** der Marker ein Lanthanid ist, vorzugsweise ausgewählt aus der Gruppe, bestehend aus Tb, Eu, Sm und Dy.

17. Nanopartikel gemäß Anspruch 13, **dadurch gekennzeichnet, dass** einer oder mehrere der Typen von Untereinheiten eine oder mehrere dritte genetisch fusionierte Bindungsgruppierungen pro Typ von Untereinheit hat/haben, wobei die Bindungsgruppierung dem Äußeren des Partikels zum Binden an einen festen Träger zugewandt ist.

18. Nanopartikel gemäß Anspruch 14, **dadurch gekennzeichnet, dass** eine erste Bindungsgruppierung ausgewählt ist aus der Gruppe, bestehend aus Protein A, Protein G, Protein L, Calmodulin-Bindungs-Peptid (CBP) und Biotin-Carboxyl-Carrier-Protein (BCCP).

19. Nanopartikel gemäß Anspruch 14, **dadurch gekennzeichnet, dass** eine erste Bindungsgruppierung ein Antikörper gegen eines der Mitglieder der Gruppe, bestehend aus CRP, ABO-Blutgruppenantigenen und TSH, ist.

20. Nanopartikel gemäß einem der Ansprüche 13 bis 19, **dadurch gekennzeichnet, dass** eine zweite Bindungsgruppierung eine Bindungsgruppierung ist, ausgewählt aus der Gruppe, bestehend aus Protein A, Protein G, Protein L, Calmodulin-Bindungs-Protein (CBP) und Biotin-Carboxyl-Carrier-Protein (BCCP) .

21. Nanopartikel gemäß einem der Ansprüche 13 bis 20, **dadurch gekennzeichnet, dass** eine zweite Bindungsgruppierung ein Antikörper gegen eines aus der Gruppe, bestehend aus CRP, ABO-Blutgruppenantigenen und TSH, ist.

22. Nanopartikel gemäß einem der Ansprüche 13 bis 21, **dadurch gekennzeichnet, dass** der minimale Radius des Nanopartikels von 10 bis 40 Nanometer beträgt.

23. Nanopartikel gemäß einem der Ansprüche 13 bis 22, **dadurch gekennzeichnet, dass** die Anzahl der Untereinheiten größer als 8, vorzugsweise größer als 20, ist.

24. Kit für einen Ligandenbindungs-Immunoassay, umfassend das Nanopartikel gemäß einem der Ansprüche 13 bis 23.

## Revendications

1. Utilisation d'une nanoparticule dans un dosage de bioaffinité par liaison de ligands, dans laquelle ladite nanoparticule comprend une enveloppe auto-assemblée formée de plusieurs sous-unités protéiniques et/ou peptidiques, sous-unités protéiniques et/ou peptidiques qui peuvent être d'un ou de plusieurs types différents, assemblées de manière organisée pour former l'enveloppe ayant une surface interne tournée vers l'intérieur et une surface externe tournée vers l'extérieur de ladite particule dans laquelle l'enveloppe de la nanoparticule est une particule d'apoferritine recombinée ou une particule de protéine Dpr/Dps analogue à l'apoferritine recombinée, dans laquelle :
a) un ou plusieurs des types de sous-unités présentent une ou plusieurs premières fractions de liaison génétiquement fusionnées par type de sous-unité, la fraction de liaison étant tournée vers l'extérieur de la particule pour lier toute protéine se liant à un ligand spécifique ; et
b)
i) la particule contient, à l'intérieur de son enveloppe, un marqueur et/ou
ii) un ou plusieurs des types de sous-unités présentent une ou plusieurs deuxièmes fractions de liaison génétiquement fusionnées par type de sous-unité, la fraction de liaison étant tournée vers l'intérieur et/ou l'extérieur de la particule liant un marqueur ; et
c) le ou les marqueurs permettent la détection de la particule ;

2. Utilisation selon la revendication 1, **caractérisée en ce que** l'enveloppe de la nanoparticule est une protéine Dpr/Dps choisie dans le groupe constitué des protéines Dpr/Dps produites par *Streptococci, Listeria, Helicobacter* et *Escherichia.*

3. Utilisation selon la revendication 1 ou 2, **caractérisée en ce que** le marqueur est choisi dans le groupe constitué d'une enzyme, d'une protéine luminescente, d'une protéine fluorescente ou colorée ou d'une molécule organique et d'un métal des terres rares.

4. Utilisation selon la revendication 3, **caractérisée en ce que** le marqueur est une protéine, de préférence une enzyme choisie dans le groupe constitué de la luciférase, GAO et GFP.

5. Utilisation selon la revendication 3, **caractérisée en ce que** le marqueur est un lanthanide choisi de préférence dans le groupe constitué de Tb, Eu, Sm et Dy.

6. Utilisation selon l'une quelconque des revendications précédentes, **caractérisée en ce qu'**un ou plusieurs des types de sous-unités présentent une ou plusieurs troisièmes fractions de liaison génétiquement fusionnées par type de sous-unité, la fraction de liaison étant tournée vers l'extérieur de la particule pour se lier à un support solide.

7. Utilisation selon l'une quelconque des revendications précédentes, **caractérisée en ce qu'**une première fraction de liaison est choisie dans le groupe constitué de la protéine A, de la protéine G, de la protéine L, de peptide liant la calmoduline (CBP) et de protéine porteuse de biotine carboxyle (BCCP).

8. Utilisation selon l'une quelconque des revendications précédentes, **caractérisée en ce qu'**une première fraction de liaison est un anticorps dirigé contre un des éléments du groupe constitué de CRP, des antigènes des groupes sanguins ABO et de TSH.

9. Utilisation selon l'une quelconque des revendications précédentes, **caractérisée en ce qu'**une deuxième fraction de liaison est une fraction de liaison choisie dans le groupe constitué de la protéine A, de la protéine G, de la protéine L, de peptide liant la calmoduline (CBP) et de protéine porteuse de biotine carboxyle (BCCP).

10. Utilisation selon l'une quelconque des revendications précédentes, **caractérisée en ce qu'**une deuxième fraction de liaison est un anticorps dirigé contre un élément du groupe constitué de CRP, des antigènes des groupes sanguins ABO et de TSH.

11. Utilisation selon l'une quelconque des revendications précédentes, **caractérisée en ce que** le rayon minimum de la nanoparticule va de 10 à 40 nm.

12. Utilisation selon l'une quelconque des revendications précédentes, **caractérisée en ce que** le nombre de sous-unités est supérieur à 8, de préférence supérieur à 20.

13. Nanoparticule, utile pour des dosages de bioaffinité par liaison de ligands, comprenant une enveloppe auto-assemblée formée de plusieurs sous-unités protéiniques et/ou peptidiques, sous-unités protéiniques et/ou peptidiques qui peuvent être d'un ou plusieurs types différents, assemblées de manière organisée pour former l'enveloppe ayant une surface interne tournée vers l'intérieur et une surface externe tournée vers l'extérieur de ladite particule dans laquelle l'enveloppe de la nanoparticule est une particule d'apoferritine recombinée ou une particule de protéine Dpr/Dps analogue à l'apoferritine recombinée, dans laquelle :
a) un ou plusieurs des types de sous-unités présentent une ou plusieurs premières fractions de liaison génétiquement fusionnées par type de sous-unité, la fraction de liaison étant tournée vers l'extérieur de la particule pour lier toute protéine se liant à un ligand spécifique ; et
b)
i) la particule contient, à l'intérieur de son enveloppe, un marqueur choisi dans le groupe constitué d'une enzyme, une protéine luminescente, une protéine fluorescente ou colorée ou une molécule organique, et un métal des terres rares et/ou
ii) un ou plusieurs des types de sous-unités possèdent une ou plusieurs deuxièmes fractions de liaison génétiquement fusionnées par type de sous-unité, la fraction de liaison étant tournée vers l'intérieur et/ou l'extérieur de la particule liant un marqueur choisi dans le groupe constitué d'une enzyme, une protéine luminescente, une protéine fluorescente ou colorée ou une molécule organique, et un métal des terres rares ; et
c) le ou les marqueurs permettent une détection de la particule ;

14. Nanoparticule selon la revendication 13, **caractérisée en ce que** les premières fractions de liaison sont choisies dans le groupe constitué des anticorps monoclonaux, des polypeptides, des récepteurs, des anticorps recombinés ou des fragments d'anticorps, des aptamères, des protéines génétiquement modifiées, et leurs dérivés.

15. Nanoparticule selon la revendication 14, **caractérisée en ce que** le marqueur est une protéine, de préférence une enzyme choisie dans le groupe constitué de la luciférase, GAO et GFP.

16. Nanoparticule selon la revendication 13, **caractérisée en ce que** le marqueur est un lanthanide, choisi de préférence dans le groupe constitué de Tb, Eu, Sm et Dy.

17. Nanoparticule selon la revendication 13, **caractérisée en ce qu'**un ou plusieurs des types de sous-unités possèdent une ou plusieurs troisièmes fractions de liaison génétiquement fusionnées par type de sous-unité, la fraction de liaison étant tournée vers l'extérieur de la particule pour se lier à un support solide.

18. Nanoparticule selon la revendication 14, **caractérisée en ce qu'**une première fraction de liaison est choisie dans le groupe constitué de la protéine A, de la protéine G, de la protéine L, du peptide liant la calmoduline (CBP) et de la protéine porteuse de biotine carboxyle (BCCP).

19. Nanoparticule selon la revendication 14, **caractérisée en ce qu'**une première fraction de liaison est un anticorps dirigé contre un des éléments du groupe constitué de CRP, des antigènes des groupes sanguins ABO et de TSH.

20. Nanoparticule selon l'une quelconque des revendications 13 à 19, **caractérisée en ce qu'**une deuxième fraction de liaison est une fraction de liaison choisie dans le groupe constitué de la protéine A, de la protéine G, de la protéine L, de la protéine liant la calmoduline (CBP) et de la protéine porteuse de biotine carboxyle (BCCP).

21. Nanoparticule selon l'une quelconque des revendications 13 à 20, **caractérisée en ce qu'**une deuxième fraction de liaison est un anticorps dirigé contre un élément du groupe constitué de CRP, des antigènes des groupes sanguins ABO et de TSH.

22. Nanoparticule selon l'une quelconque des revendications 13 à 21, **caractérisée en ce que** le rayon minimum de la nanoparticule va de 10 à 40 nm.

23. Nanoparticule selon l'une quelconque des revendications 13 à 22, **caractérisée en ce que** le nombre de sous-unités est supérieur à 8, de préférence supérieur à 20.

24. Trousse pour un dosage immunologique par liaison de ligands comprenant la nanoparticule selon l'une quelconque des revendications 13 à 23.
